(19) Europäisches Patentamt
European Patent Office
Office européen des brevets

(11) Veröffentlichungsnummer: **0 336 349**
**A2**

# (12) EUROPÄISCHE PATENTANMELDUNG

(21) Anmeldenummer: **89105840.6**

(22) Anmeldetag: **04.04.89**

(51) Int. Cl.[4]: **C07D 309/10 , A61K 31/35**

Der Anmelder hat eine Erklärung nach Regel 28 (4) EPÜ (Herausgabe einer Probe nur an einen Sachverständigen) eingereicht. Eingangsnummer(n) derHinterlegung(en): DSM 4249.

Patentansprüche für folgende Vertragsstaaten: ES + GR.

(30) Priorität: **06.04.88 DE 3811463**

(43) Veröffentlichungstag der Anmeldung: **11.10.89 Patentblatt 89/41**

(84) Benannte Vertragsstaaten: **AT BE CH DE ES FR GB GR IT LI LU NL SE**

(71) Anmelder: **BEHRINGWERKE Aktiengesellschaft**
**Postfach 1140**
**D-3550 Marburg 1(DE)**

(72) Erfinder: **Kraemer, Hans Peter, Dr.**
**Birkenweg 16**
**D-3550 Marburg(DE)**
Erfinder: **Robbel, Lutz, Dr.**
**Höhenweg 72a**
**D-3550 Marburg(DE)**
Erfinder: **Schwenen, Ludger Michael, Dr.**
**Karl-Müller-Strasse 9**
**D-3550 Marburg(DE)**

(74) Vertreter: **Meyer-Dulheuer, Karl-Hermann, Dr. et al**
**HOECHST Aktiengesellschaft Postfach 3540**
**D-6121 Wiesbaden(DE)**

(54) **Sakyomicin E und dessen Derivate, Verfahren zu ihrer Herstellung und ihre Verwendung.**

(57) Es werden die Verbindungen der Formel I

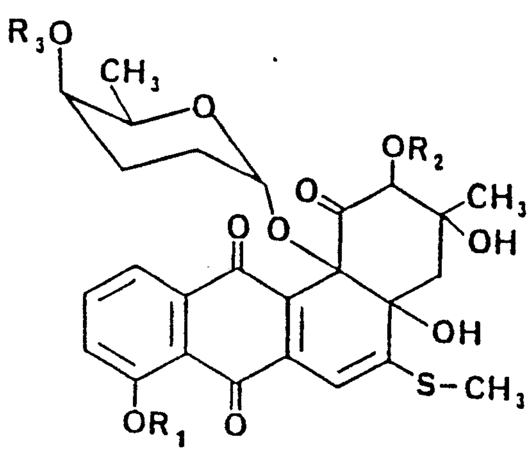

I

worin $R_1$ bis $R_3$ unabhängig voneinander Wasserstoff oder eine $(C_1-C_{18})$-Acylgruppe bedeuten, sowie ein Verfahren zu ihrer Herstellung beschrieben. Diese Verbindungen besitzen tumorhemmende Eigenschaften.

EP 0 336 349 A2

## Sakyomicin E und dessen Derivate, Verfahren zu ihrer Herstellung und ihre Verwendung

Die Erfindung betrifft Sakyomicin E und Derivate von diesem mit der allgemeinen Formel I

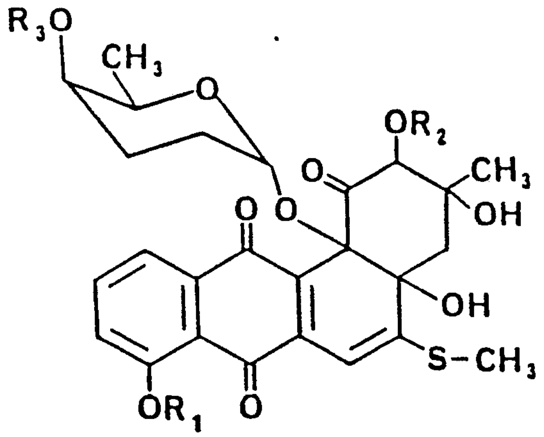

I

in der $R_1$-$R_3$ unabhängig voneinander Wasserstoff oder eine $(C_1$-$C_{18})$-Acylgruppe bedeuten, ein Verfahren zu ihrer Herstellung, das dadurch gekennzeichnet ist, daß man Actinomadura spec. fermentiert, die Verbindungen der allgemeinen Formeln I aus dem Kulturmedium oder Mycel isoliert und gegebenenfalls acyliert, sowie ihre Verwendung zur Herstellung von Heilmitteln.

Sakyomicine werden durch Fermentation mit einem Mikroorganismenstamm (hinterlegt am 15. Sept. 1987 bei der deutschen Sammlung für Mikroorganismen (DSM), 3300 Braunschweig, Mascheroder Weg 1b unter der Hinterlegungsnummer DSM 4249), der aus einer Bodenprobe aus Indien isoliert wurde, hergestellt. Der Mikroorganismus wurde als Saccaromonospora spec. identifiziert (vormals fälschlich als Actinomadura spec. bezeichnet). Aus diesen Sakyomicinen kann man Sakyomicinone durch saure Hydrolyse oder Methanolyse erhalten.

Die bekannten Sakyomicine und Sakyomicinone wirken antibakteriell und tumorhemmend und können daher in Form pharmazeutischer Präparate zur Behandlung von bakteriell verursachten Infektionen oder zur Behandlung bei Tumorerkrankungen an Mensch und Tier verwendet werden.

Im folgenden wird die Erfindung, insbesondere mit ihren bevorzugten Ausgestaltungen, erläutert und in den Patentansprüchen definiert.

Der Mikroorganismus ist wie folgt charakterisiert:

| | |
|---|---|
| Sporenoberfläche: | smooth |
| Sporenmorphologie: | RF |
| Luftmycelfarbe: | weiß - grau |
| Substratmycelfarbe: | dunkelrot |

Für die Fermentation wird der Stamm DSM 4249 eingesetzt.

Als bevorzugte Kohlenstoffquelle für die aerobe Fermentation eignet sich besonders Glucose, sowie kohlenhydrathaltige Naturprodukte wie Malzextrakt. Als bevorzugte stickstoffhaltige Nährstoffquelle kommen Pepton und Trypton, sowie Fleischextrakt, Hefeextrakt in Betracht. Außerdem kann die Nährlösung beispielsweise Chloride, Carbonate oder Phosphate als zusätzliche anorganische Salze oder andere Spurenelemente enthalten.

Die Konzentration an Sakyomicin E ist von den Kulturbedingungen des Mikroorganismus abhängig. Bei Verwendung eines Nährmediums, das Soja-Pepton (10 g/l), Glucose (20 g/l), $CaCO_3$ (2 g/l) und $CoCl_2.6H_2O$ (1 mg/l) bei einem pH von 7.2 enthält, wird Sakyomicin E neben dem bekannten Sakyomicin A zu etwa 20 % gebildet. Wird dem Medium die Aminosäure Methionin (bis zu 200 mg/l) zugesetzt, so werden Sakyomicin E und ein weiteres Sakyomicin als Hauptprodukte gebildet (80 %). Ebenso ist das Mengenverhältnis der Sakyomicine beim Zusatz anderer schwefelhaltiger Aminosäuren zur Seite des Sakyomicin E zu verschieben.

Die Fermentation verläuft aerob, also beispielsweise submers unter Schütteln oder Rühren in Schüttelkolben oder Fermentern, gegebenenfalls unter Einführung von Luft oder Sauerstoff. Die Fermentation kann vorzugsweise bei einer Temperatur von 25 bis 30 °C erfolgen. Man kultiviert den Mikroorganismus etwa 65

bis 70 Stunden unter den genannten Bedingungen.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise in einem Volumenverhältnis 1:14, überimpft werden. Die Vorkultur erhält man beispielsweise, indem man ein versportes Luftmycel in eine Nährlösung uberimpft und etwa 65 - 70 Stunden wachsen läßt. Das versporte Mycel kann erhalten werden, indem man den Stamm etwa 10 Tage auf einem Nährboden, beispielsweise Glucose-Hefextrakt-Malzextrakt-Agar, wachsen läßt.

Der Fermentationsverlauf kann anhand des pH-Wertes und des $pO_2$-Wertes der Kultur oder des Mycelvolumens, durch Dünnschichtchromatographie (DC) oder Extraktion mit anschließender UV-Messung bei 416 nm oder durch HochdruckFlüssigkeits-Chromatographie (HPLC) überwacht werden.

Die Isolierung von Sakyomicin E aus dem Kulturmedium erfolgt nach bekannten Methoden unter Berücksichtigung der chemischen, physikalischen und biologischen Eigenschaften des Produktes. Zum Testen der Antibiotika-Konzentration im Kulturmedium oder in den einzelnen Isolierungsstufen kann die DC, beispielsweise auf Kieselgel mit Chloroform/Methanol als Laufmittel, verwendet werden, wobei die Menge der gebildeten Antibiotika zweckmäßig mit einer Eichlösung verglichen wird.

Sakyomicin E liegt zusammen mit dem bekannten Sakyomicin A im Mycel bzw. im Kulturfiltrat vor. Die Antibiotika können aus der unfiltrierten Kulturbrühe mit einem mit Wasser nicht oder nur wenig mischbaren, organischen Lösungsmittel wie Ethylacetat oder Chloroform extrahiert werden. Da sich jedoch nur ein geringer Teil der Verbindungen im Mycel befindet, ist es vorteilhaft, die Kulturbrühe vom Mycel zu trennen, beispielsweise durch Zentrifugation oder durch Filtrieren, vorzugsweise unter Zugabe von Filterhilfsmitteln.

Ebenso ist es möglich, Sakyomicin E aus der Kulturbrühe mit Hilfe von Sorbentien zu adsorbieren (z.B. ®XAD-2 oder ®XAD-4, Fa. Serva) und anschließend mit Hilfe steigender Methanolkonzentration vom Adsorberharz zu eluieren.

Die Antibiotika können dann aus dem Überstand bzw. Filtrat isoliert werden, zweckmäßig in leicht saurem bis neutralem pH-Bereich, vorzugsweise bei pH 6 - 7. Dazu verwendet man zweckmäßig mit Wasser wenig oder nicht mischbare organische Lösemittel, insbesondere Ethylacetat oder chlorierte Kohlenwasserstoffe wie Chloroform oder Methylenchlorid. Die farbigen Extrakte werden, gegebenenfalls nach Eindampfen und Aufnehmen in einem polaren Lösungsmittel, durch Fällen mit einem unpolaren Lösungsmittel, zweckmäßig einem Kohlenwasserstoff wie n-Hexan oder einem Gemisch wie Petrolether, von stark lipophilen Begleitstoffen befreit, die einen Großteil des Gesamtextraktes ausmachen können. Aus dem Rückstand kann Sakyomicin E durch Chromatographie isoliert werden.

Zur Isolierung des Sakyomicin E aus dem entfetteten Rohextrakt wird dieser zweckmäßig über Kieselgel (Säule oder Präparative DC) gereinigt, wobei sich als Fließmittel ein Gemisch aus niedermolekularen Chlorkohlenwasserstoffen und Alkoholen, beispielsweise Chloroform und Methanol im Volumenverhältnis 9:1 oder Methylenchlorid und Ethanol im Volumenverhältnis 9:1, bewährt haben. Die in Form verschiedenfarbiger Zonen adsorbierten Komponenten werden nacheinander in Form verschiedenfarbiger Fraktionen eluiert. Die Hauptfraktion enthält Sakyomicin E.

Zur Isolierung des reinen Sakyomycin E können die üblichen Verfahrensschritte Verwendung finden, wie Chromatographie, Gelfiltration oder Fällung aus seinen Lösungen in geeigneten organischen Lösungsmitteln. Besonders bewährt hat sich eine Chromatographie an Kieselgel, wobei als Laufmittel eine Mischung aus einem niederen Alkohol und einem Halogenkohlenwasserstoff, beispielsweise Chloroform/Methanol im Volumenverhältnis 9:1 Verwendung findet, gefolgt von einer HPLC-Trennung an einer Reversed-Phase C-18 Säule mit einem isocratischen Laufmittel oder einem Gradienten aus einem niederen Alkohol und Wasser, beispielsweise Methanol/Wasser im Verhältnis 1:1 (v/v) oder Acetonitril und Wasser im Verhältnis 2:3 (v/v) oder einem Gradienten, der von 100 % bis 0% Wasser, vorzugsweise 40 %, kontinuierlich auf 100 % des organischen Lösungsmittels läuft, mit einer Anstiegsgeschwindigkeit von 1 bis 10 % pro Minute, vorzugsweise 2 % pro Minute. Das Sakyomicin E wird dann aus der entsprechenden Fraktion durch Verdampfen der organischen Komponente aus dem Laufmittel oder unter Zugabe von 50 % Wasser und anschließende Extraktion mit schließende Extraktion mit einem organischen Lösungsmittel, z.B. Ethylacetat und anschließendes Ausfällen, beispielsweise durch Eintropfen einer konzentrierten Lösung von Sakyomicin E in ein unpolares Lösungsmittel, insbesondere einen Kohlenwasserstoff, wie n-Hexan oder Petrolether, gewonnen.

Sakyomicin E ist ein roter, amorpher Farbstoff, der gut in Ethylacetat, Methanol, Aceton, DMSO und Chloroform löslich, jedoch nicht oder nur sehr gering löslich in Alkanen ist. Die Substanz ist im festen Zustand wie auch als Lösung im pH-Bereich von 3 bis 9, insbesondere von 5 bis 8, stabil und läßt sich somit in übliche galenische Zubereitungen einarbeiten.

Sakyomicin E zeigt gegen gram-positive wie gram-negative Bakterien eine antibakterielle Wirkung, die beispielsweise im Platten-Agardiffusions-Test in vitro (10 µl/Testrondel, 6mm Durchmesser) ermittelt werden kann. Verschiedene Keime, die sich gegenüber einer Hemmkonzentration von größer als 10 µg/ml sensitiv

zeigen sind beispielsweise: E. coli, Staph. aureus, Strep. faecalis.

Die tumorhemmende Wirkung von Sakyomicin E konnte an menschlichen und tierischen Tumorzellen in vitro gezeigt werden.

Die Überführung des Sakyomicin E in die entsprechenden ($C_1$-$C_{18}$)-Acylverbindungen, bevorzugt ($C_1$-$C_{10}$)-Acylverbindungen, insbesondere die Acetylverbindungen, erfolgt nach an sich bekannter Weise mit einem Acetylierungsmittel, das die gewünschten Acylreste einer organischen Carbonsäure einführt. Dabei verwendet man die entsprechende Carbonsäure oder ein reaktionsfähiges Derivat, insbesondere ein Anhydrid. Die Acylierung kann in Gegenwart geeigneter Kondensationsmittel, bei Verwendung von freien Carbonsäuren, z.B. in Gegenwart von Carbodiimidverbindungen, wie Dicyclohexylcarbodiimid oder Carbonylverbindungen, wie Diimidazolylcarbonyl und bei Verwendung von reaktionsfähigen Carbonsäurederivaten z.B. in Gegenwart von basischen Mitteln, wie Triniederalkylamin, z.B. Triethylamin oder heterocyclischen Basen, z.B. Pyridin oder 4-Dimethylaminopyridin, oder basischen Salzen, z.B. wasserfreiem Natriumacetat, durchgeführt werden. Die Acylierungsreaktion kann in Abwesenheit oder in Gegenwart eines Lösungsmittels oder Lösungsmittelgemisches unter Kühlen, bei Raumtemperatur oder unter Erwärmen und, wenn notwendig, in einem abgeschlossenen Gefäß und/oder in einer Inertgas-, z.B. Stickstoffatmosphäre durchgeführt werden. Geeignete Lösungsmittel sind beispielsweise einfache oder substituierte, z.B. chlorierte, aliphatische, cycloaliphatische oder aromatische Kohlenwasserstoffe, wobei man geeignete Veresterungsagentien, wie Acetanhydrid, aber auch geeignete Basen, wie Pyridin, als Verdünnungsmittel verwenden kann.

Bevorzugt wird die Reaktion jedoch mit einem Anhydrid, insbesondere Acetanhydrid, in Gegenwart von Pyridin oder Natriumacetat durchgeführt. Die Reationstemperaturen und Reaktonszeiten liegen zwischen -10 und 100 °C und 2 Minuten und 48 Stunden, bevorzugt zwischen 20 und 30 °C und 8 Minuten und 16 Stunden. Das Verhältnis Anhydrid zur Base liegt im Bereich von 20:1 bis 1:1, bevorzugt 2:1. Die Konzentration von Sakyomicin E im Reaktionsansatz liegt zwischen 0,05 bis 10 %, bevorzugt bei 0,1 bis 1 %.

Nach Beendigung der Reaktion kann das Reaktionsprodukt durch Extraktion isoliert werden. Zur weiteren Reinigung wird es dann beispielsweise an Kieselgel chromatographiert.

Alternativ können Mono- und Diacylverbindungen durch basische Spaltung von 2,8,4′-Tri-O-acyl-sakyomicin E hergestellt werden. Verwendung finden Basen wie die Alkali- und Erdalkalihydroxide oder Alkalicarbonate in wäßriger/alkoholischer Lösung bei -10 bis 100 °C. Die Acylspaltung wird bevorzugt mit einer gesättigten Natriumcarbonatlösung in wäßrig/methanolischer Lösung bei 25 °C durchgeführt. Die anschließende Aufarbeitung erfolgt wie oben erwähnt.

Die genannten Acylverbindungen leiten sich von organischen Carbonsäuren ab. Diese enthalten geradkettige, verzweigte oder cyclische aliphatische, aliphatisch-aromatisch oder aromatische Kohlenwasserstoffreste, die unsubstituiert oder durch ein Halogen, z.B. Chlor oder Brom, oder veresterte oder veretherte Hydroxygruppen substituiert sind.

Die so hergestellten Verbindungen zeigen ebenfalls tumorhemmende Wirkung.

Im folgenden wird die Erfindung anhand von Beispielen erläutert. Prozentangaben beziehen sich, wenn nicht anders angegeben, auf das Gewicht.

## Beispiele

Beschreibung des Stamms DSM 4249:

Morphologische Charakteristika:

Für die Auswertungen wurden die Arbeiten von Shirling, E.B. und Gottlieb, D.: Methods for characterization of Streptomyces species, Int. J. Syst. Bacteriol. 16, 313 -340, 1966 verwendet. Die Untersuchungen für die Licht-und Elektronenmikroskopie wurden an einer 14 Tage alten Kultur durchgeführt.

Physiologische Charakteristika:

| Kohlenhydratverwertung | |
|---|---|
| Glycerin | +/- |
| D - Xylose | + |
| Lactose | - |
| D - Fructose | + |
| L - Rhamnose | + |
| Maltose | - |
| Inulin | + |
| Inositol | + |
| Raffinose | + |
| D - Galactose | + |
| L - Arabinose | + |
| Mannitol | + |
| D - Mannose | + |
| Sucrose | - |
| Spaltung von Harnstoff | |
| Urease Aktivität | + |
| Melaninbildung | - |
| Nitratreduktion | - |
| Hämolytische Aktivität | - |

1a) Herstellung einer Sporensuspension des Produzentenstammes:

| Zusammensetzung des Vorkultur-Mediums: | |
|---|---|
| Sojabohnenpepton | 10 g |
| Glucose | 20 g |
| $CaCO_3$ | 2 g |
| $CoCl_2.6\ H_2O$ | 1 mg |
| Leitungswasser | 1 l |
| pH-Wert (nach dem Autoklavieren) | 7,2 |

| Zusammensetzung des Agars: | |
|---|---|
| Glucose | 20 g |
| Malzextrakt | 10 g |
| Hefeextrakt | 4 g |
| $CaCO_3$ | 2 g |
| Agar | 12 g |
| Leitungswasser | 1 l |
| pH-Wert | 7,2 |
| (25 ml Nährlösung/Petrischale Ø | 9 cm) |

Tabelle 1:

| Medium | Luftmycel | Wachstum | Substratmycel | Lösl. Pigment |
|---|---|---|---|---|
| Glucose-Hefeextrakt-Malzextrakt Agar | weiß (grau-schimmernd) | + + + | dunkelrot | + (dunkelrot) |
| Glycerin-Asparagin Agar | gelblich-weiß | + + + | bräunlich-rot | + (bräunl.-rot) |
| Stärke-Mineralsalz Agar | weiß, spärlich ausgebildet | + + | beige | - (leicht rosa) |
| Glycerin-Glycin Agar | weiß-grau | + + + | beige-rot | + (dunkelrot) |
| Glycerin-Stärke-Glutamat-Agar | steingrau | + + + | rot-braun | + (violett) |
| Kartoffel-Karotten Agar | weiß | + + + | beige | - |
| Malzextrat Agar | weiß-grau | + + + | rot | + (orangerot) |
| Casein-Stärke-Pepton Hefeextrakt Agar | weiß, spärlich ausgebildet | + + | beige | - (leicht rosa) |
| Czapek-Pepton-Hefe Agar | grau-weiß, spärlich ausgebildet | + + | beige | - (leicht rosa) |

200 ml Nährlösung des Vorkulturmediums in einem Erlenmeyerkolben werden mit dem Stamm DSM 4249 beimpft und 68 Stunden bei +27° C und 200 UpM auf der rotierenden Schüttelmaschine inkubiert. Anschließend wird 1 ml Kulturflüssigkeit auf dem Nährboden gleichmäßig verteilt und 14 Tage bei +27° C inkubiert. Die nach dieser Zeit auf einer Agarplatte entstandenen Sporen werden mit einem Glasspatel entnommen und in einem Glasmörser, (Braun Melsungen) mit Zusatz von 12 ml physiologischer NaCl homogenisiert.

b) Herstellung einer Kultur bzw. einer Vorkultur des Produzentenstammes in Erlenmeyerkolben

Ein 1000 ml-Erlenmeyerkolben mit 300 ml Nährlösung (beschrieben unter Vorkulturmedium) wird mit 6 ml der Sporensuspension angeimpft und auf einer Schüttelmaschine bei 200 UpM und +27° C inkubiert.

Sakyomicine sind nach ca. 50 Stunden nachweisbar. Eine optimale Sakyomicin E Produktion wird durch Zugabe von 200 mg/l der Aminosäure Methionin und nach ca. 60 Stunden erreicht.

c) Herstellung einer Fermentvorkultur

Ein 500 ml Erlenmeyerkolben mit 100 ml Nährlösung (beschrieben unter Vorkulturmedium) wird mit Sporenmaterial einer Agarplatte angeimpft und auf einem Inkubationsschüttler bei 200 UpM und +27° C 72 Stunden inkubiert.

2. Herstellung von Sakyomicin E

| Zusammensetzung des Fermentermediums: | |
|---|---|
| Sojabohnenpepton | 10 g |
| Glucose | 20 g |
| $CaCO_3$ | 2 g |
| $CoCl_2.6\ H_2O$ | 1 mg |
| Methionin | 200 mg |
| Leitungswasser | 1 l |
| pH-Wert (nach dem Autoklavieren) | 7,2 |

Ein 10 l Fermenter wird unter folgenden Bedingungen betrieben:

| | |
|---|---|
| Inkubationszeit | 72 - 96 Stunden |
| Inkubationstemperatur | + 27° C |
| Rührgeschwindigkeit | 200 - 300 rpm |
| Belüftung | submers 300 - 400 l/Std. |

Durch wiederholte Zugabe von einigen Tropfen Polypropylenglykol kann die Schaumentwicklung unterdrückt werden. Das Produktionsmaximum liegt bei ca. 80 Stunden. Der pH-Wert beträgt am Ende ca. 7.8. Die Ausbeuten liegen bei ca. 1.7 g Rohextrakt/10 l Kulturbrühe.

3. Isolierung von Sakyomicin E

Nach der Fermentation des Stamms DSM 4249 wird die Kulturbrühe unter Zusatz von 2 % Celite als Filterhilfsmittel filtriert. Das Mycel wird mit Aceton extrahiert, die organische Phase eingedampft und der wäßrige Rückstand mit Ethylacetat extrahiert. Das Kulturfiltrat wird ebenfalls bei pH 7.5 erschöpfend mit Ethylacetat extrahiert. Dieser Extrakt wird mit dem Mycel vereinigt (oder getrennt aufgearbeitet), getrocknet und eingedampft. Der ölige Rückstand wird in wenig Chloroform aufgenommen und in reichlich Petrolether oder n-Hexan getropft. Der ausgefallene Niederschlag wird abzentrifugiert und getrocknet. Das Rohprodukt wird an einer Kieselgelsäule (®LiChroprep Si100, 25-40µm, Fa. Merck) mit Chloroform/Methanol (9:1 v/v) gereinigt. In den Fraktionen 3 und 4 (rot) des Eluats war Sakyomicin E angereichert. Es wurde mit Hilfe der HPLC an RP 18 Säulen mit Methanol/Wässer 1:1 nachgereinigt.

4. Reinigung von Sakyomicin E

Die Reinigung des Sakyomicin E auf über 98 % Reinheit erfolgt mit Hilfe der Reversed Phase HPLC. Hierzu werden 60 mg auf eine semipräparative HPLC-Säule Nucleosil 7 C18 der Fa. Macherey & Nagel, FRG, gegeben und mit einem Eluenten von 50 % Methanol/Wasser getrennt. Die Flußgeschwindigkeit beträgt 9 ml/min. Die Detektion erfolgt mit Hilfe eines UV-Detektors bei einer Wellenlänge von 260 nm. Zur entsprechenden Fraktion wird Wasser (ca. 50 %) hinzugegeben. Danach wird einmal mit der gleichen, anschließende zweimal mit der Hälfte an Ethylacetat extrahiert. Nach Trocknung mit $Na_2SO_4$ wird das organische Lösungsmittel im Vakuum verdampft. Durch Aufnahme in geringer Menge an Chloroform und Ausfällen in n-Hexan erhält man sauberes, kristallines Sakyomicin E.

5. Charakterisierung von Sakyomicin E

Die physikalisch-chemischen Eigenschaften des wie in Beispiel 3 isolierten Antibiotikums Sakyomicin E sind wie folgt:
Dünnschichtchromatographie (Alufolie Kieselgel 60 F254, Fa. Merck und Alugram Sil G, Fa. Macherey & Nagel):

| | Fa. Merck | Fa. Macherey & Nagel |
|---|---|---|
| Chloroform/Methanol (9:1) | Rf 0.34 | Rf 0.49 |
| Benzol/Methanol (8:2) | Rf 0.32 | Rf 0.43 |
| Ethylacetat/Methanol(98:2) | Rf 0.42 | Rf 0.54 |
| Dichlormethan/Ethanol (9:1) | Rf 0.48 | Rf 0.56 |

$C_{26}H_{28}O_{10}$ (MW 532.57)
Schmp.: 204° C
IR (KBr-Preßling): Fig. 1
UV (in Methanol): Fig. 2
$^1$H-NMR (200 MHz, $CDCL_3$): Fig. 3

6. Herstellung von 2,8,4′-Tri-O-acetylsakyomicin E

40 mg Sakyomicin E werden in 40 ml Acetanhydrid/Pyridin Gemisch (2:1, v/v) 17 Stunden bei Raumtemperatur gerührt. Man gießt das Gemisch auf Eiswasser (ca. 10 ml) und extrahiert zweimal mit 20 ml Chloroform. Man engt die organische Phase im Vakuum ein und entfernt das restliche Pyridin durch mehrfaches Aufnehmen und Eindampfen mit Toluol. Den Rückstand chromatographiert man an Kieselgel 60 (präparative DC Kieselgel 60, 0.5 mm Schichtdicke, Fa. Merck) im Laufmittel Chloroform/Aceton (10:3, v/v). Man erhält zwei Hauptbanden.

1. Obere rote Bande (Rf = 0.62) Tetra-O-acetyl-Sakyomicin E 8 mg
2. Untere rote Bande (Rf = 0.38) Tri-O-acetyl-Sakyomicin E 42 mg

$C_{32}H_{34}O_{13}$ (MW 658.68)
Schmp.: 177 °C
IR (KBr-Preßling): Fig. 4
UV (in Methanol): Fig. 5
$^1$H-NMR (200 MHz, $CDCl_3$): Fig. 6

7. In vitro Testung auf cytotoxische Aktivität

Die cytotoxische Aktivität der erfindungsgemäßen Verbindungen wurde in zwei unterschiedlichen in vitro-Testsystemen ermittelt.

1. Proliferationstest (MTT-assay)

In diesem Testsystem wird nach 3tägiger Inkubation mit der Testsubstanz die Anzahl lebender bzw. toter Zellen anhand der nur in lebenden Zellen auftretenden NADP-abhängigen Reduktion eines farblosen Substrates zu einem tiefrot gefärbten Endprodukt gemessen.

L1210 A 549 oder HT 29 in exponentieller Wachstumsphase werden in einer Zelldichte von 5 x $10^3$ Zellen/ml in RPMI 1640 in einer 96 well-Mikrotiterplatte für 72 Stunden mit unterschiedlichen Konzentrationen der Testsubstanz bei 37 °C, 5 % $CO_2$ und 95 % relativer Luftfeuchte inkubiert. Kontrollexperimente erhalten anstelle der Testsubstanz lediglich Wachstumsmedium. Für jede Testsubstanz sowie für die Kontrolle werden 4fach Bestimmungen angesetzt. Nach 65 Stunden Inkubation werden 50 µl einer MTT-Lösung (2,5 mg/ml in Phosphatgepuffertem Kochsalz) zugegeben. In Gegenwart lebender Zellen wird MTT zu einem dunkelroten unlöslichen Formazanfarbstoff reduziert. Diese Reaktion ist nach 7 Stunden (L1210 Zellen) bzw. nach 24 Stunden (A 549, HT 29 Zellen) beendet und das überstehende Medium wird sorgfältig abgesaugt. Der unlösliche Farbstoff wird durch Zugabe von 100 µl DMSO aufgelöst und die Extinktion der so entstehenden Lösung anschließend für jedes well in einem Multiscan Photometer 340 CC der Fa. Flow bei einer Wellenlänge von 492 nm vermessen.

Aus dem Verhältnis der Extinktionen behandelter und unbehandelter Zellen ergibt sich eine Dosis-Wirkungskurve, aus der die Konzentration, die gerade 50 % der Zellen ($IC_{50}$) abtötet, abgelesen werden kann. Für wiederholte Untersuchungen beträgt der Variationskoeffizient weniger als 15 %. Die Ergebnisse sind in Tabelle 1 zusammengefaßt.

2. Stammzelltest

Dieses Testsystem prüft die Fähigkeit der Testsubstanz, die Bildung von Tumorzellkolonien in Weichagar zu inhibieren. Der Test wurde entsprechend der von Hamburger und Salmon beschriebenen Methodik mit den unten beschriebenen Modifikationen durchgeführt. Konditioniertes Medium wurde durch McCoy 5A Medium ersetzt. Als Folge der hohen Klonierungsrate der Zellinien in Soft Agar wurde die Anzahl an Tumorzellen pro Platte auf 5 x $10^2$ (L1210) bzw. 5 x $10^3$ (A 549, HT 29) reduziert. Die Zellen wurden mit unterschiedlichen Konzentrationen der Testsubstanz für 1 Stunde bei 37 °C inkubiert. Anschließend wurden die Zellen zweimal mit McCoy 5A Medium gewaschen und anschließend in einem Zwei-Schichten-Agar System als obere Schicht entsprechend der Methode von Hamburger und Salmon ausplatiert. Zusätzliche Parallelexperimente wurden unter Verwendung einer kontinuierlichen Inkubationszeit durchgeführt, wobei unterschiedliche Konzentrationen der Testsubstanz der der oberen Agarschicht vor Ausplatieren der Zellen zugemischt wurden.

Die Platten wurden in einem Brutschrank mit 5 % $CO_2$, 20 % $O_2$ und 95 % relativer Luftfeuchte für 5 - 7 Tage (L1210) bzw. 14 Tage (A 549, HT 29) inkubiert. Nach dieser Zeit wurden Kolonien mit einem Durchmesser (größer) 60 µm mit Hilfe eines Bild-Analyse-Systems (FAS II, Bausch und Lomb) gezählt.

Die Ergebnisse wurden angegeben als prozentualer Anteil der Kolonien in behandelten versus unbehandelten Gruppen. Der Variationskoeffizient bei wiederholten Experimenten war kleiner als 15 %. Aus der Dosis-Wirkungskurve wurde die $IC_{50}$ für die kontinuierliche und einstündige Inkubation bestimmt (Tab. 2).

Die Daten zeigen, daß beide Verbindungen bei unterschiedlichen humanen (A 549, HT 29) bzw. murinen (L1210) Zelllinien eine hohe Zytotoxizität aufweisen.

Tabelle 2

| | $IC_{50}$ (µg/ml) | | |
|---|---|---|---|
| | L1210 | A 549 | HT 29 |
| Sakyomicin E | 0.50 | 0.30 | 0.08 |
| 2,8,4′-Tri-O-acetylsakyomicin E | 0.29 | 0.37 | 0.11 |

Tabelle 3

| | $IC_{50}$ (µg/ml) | |
|---|---|---|
| | cont. Incubation | 1 h Incubation |
| Sakyomicin E | 0.24 | 3.0 |
| 2,8,4′-Tri-O-acetylsakyomicin E | 0.30 | größer als 1.0 |

**Ansprüche**

1. Verbindung der Formel I

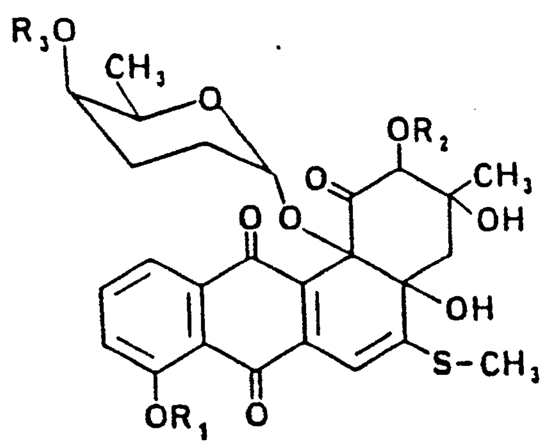

I

in der $R_1$ bis $R_3$ unabhängig voneinander Wasserstoff oder eine ($C_1$-$C_{18}$)-Acylgruppe bedeuten.

2. Verbindung nach Anspruch 1, in der $R_1$ bis $R_3$ unabhängig voneinander Wasserstoff oder Acetyl bedeutet, wobei mindestens ein R Acetyl ist.

3. Verfahren zur Herstellung der Verbindung der allgemeinen Formel I in Anspruch 1, dadurch gekennzeichnet, daß man

a) Saccaromonospora spec. DSM 4249 kultiviert,

b) die Verbindung der allgemeinen Formel I, in der $R_1$ bis $R_3$ Wasserstoff bedeuten, isoliert und gegebenfalls

c) diese Verbindung acyliert.

4. Verfahren nach Anspruch 3, dadurch gekennzeichnet, daß in einer Nährlösung mit 2 % Glucose, 1 % Sojapepton, 0,2 % Calciumcarbonat und 0.0001 % Kobaltchlorid kultiviert wird.

5. Verfahren nach Anspruch 3, dadurch gekennzeichnet, daß in einem Temperaturbereich von 15 - 38° C kultiviert wird.

6. Verfahren nach Anspruch 3, dadurch gekennzeichnet, daß in einem Temperaturbereich von 25 bis 30° C kultiviert wird.

7. Verfahren nach Anspruch 3, dadurch gekennzeichnet, daß über einen Zeitraum von 40 - 80 Stunden kultiviert wird.

8. Verfahren nach Anspruch 3, dadurch gekennzeichnet, daß 65 - 70 Stunden kultiviert wird.

9. Verbindung nach Anspruch 1 als Heilmittel.

10. Saccaromonospora spec. DSM 4249.

Patentansprüche für folgende Vertragsstaaten: ES, GR

1. Verfahren zur Herstellung der Verbindung der allgemeinen Formel I

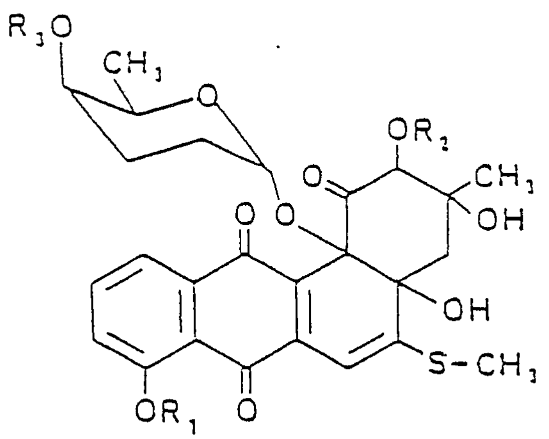

I

in der $R_1$ bis $R_3$ unabhängig voneinander Wasserstoff oder eine ($C_1$-$C_{18}$)-Acylgruppe bedeuten, dadurch gekennzeichnet, daß man

a) Actinomadura spec. DSM 4249 kultiviert,

b) die Verbindung der allgemeinen Formel I, in der $R_1$ bis $R_3$ Wasserstoff bedeuten, isoliert und gegebenenfalls

c) diese Verbindung acyliert.

2. Verfahren nach Anspruch 1, dadurch gekennzeichnet, daß in einer Nährlösung mit 2 % Glucose, 1 % Sojapepton, 0,2 % Calciumcarbonat und 0.0001 % Kobaltchlorid kultiviert wird.

3. Verfahren nach Anspruch 1, dadurch gekennzeichnet, daß in einem Temperaturbereich von 15 - 38° C kultiviert wird.

4. Verfahren nach Anspruch 1, dadurch gekennzeichnet, daß in einem Temperaturbereich von 25 bis 30° C kultiviert wird.

5. Verfahren nach Anspruch 1, dadurch gekennzeichnet, daß über einen Zeitraum von 40 - 80 Stunden kultiviert wird.

6. Verfahren nach Anspruch 1, dadurch gekennzeichnet, daß 65 - 70 Stunden kultiviert wird.

7. Actionomadura spec. DSM 4249.

FIG.1

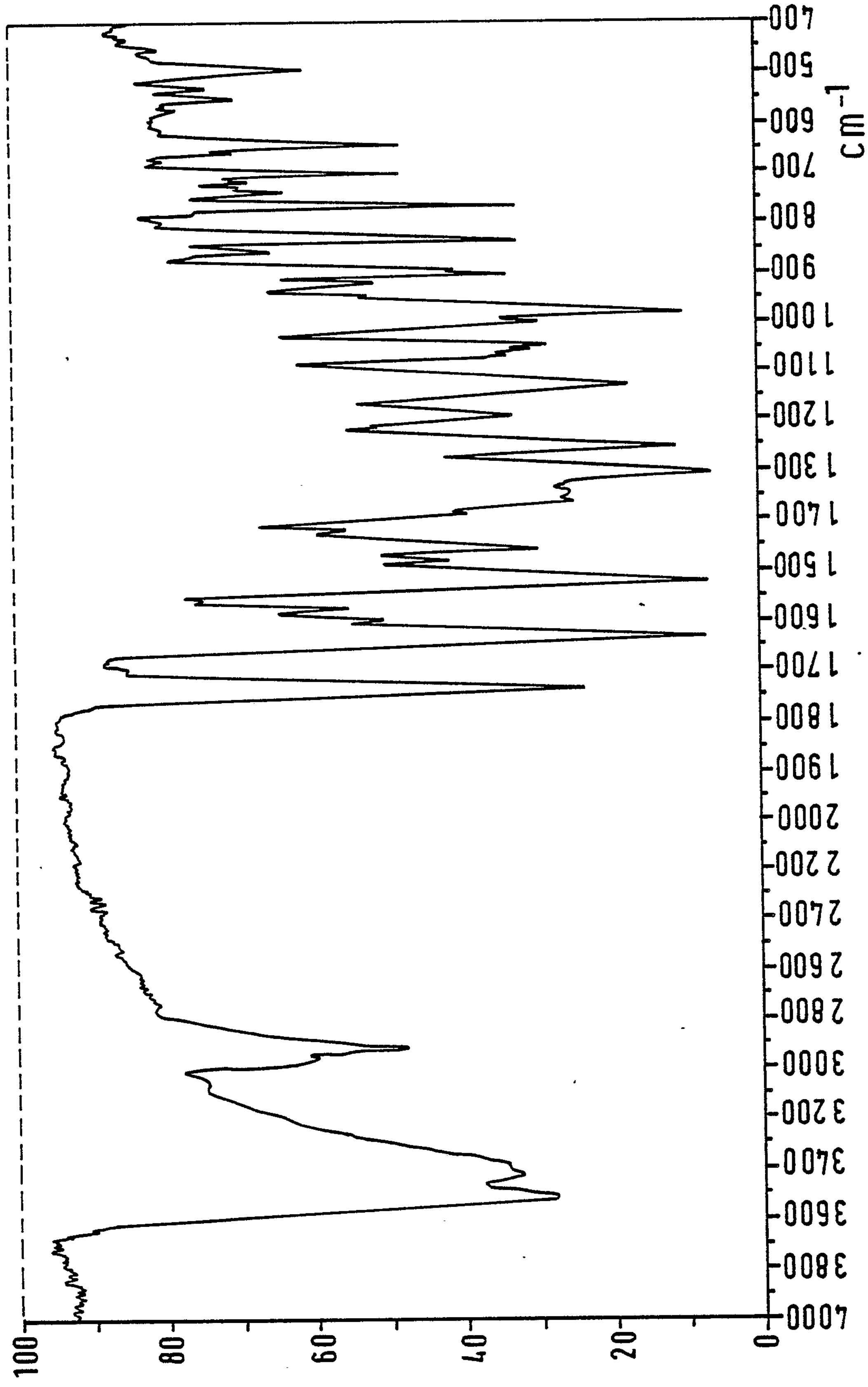
100
80
60
40
20
0
400
500
600
700
800
900
1 000
1 100
1 200
1 300
1 400
1 500
1 600
1 700
1 800
1 900
2000
2 200
2400
2 600
2 800
3000
3 200
3400
3600
3 800
4000
cm-1

FIG. 2

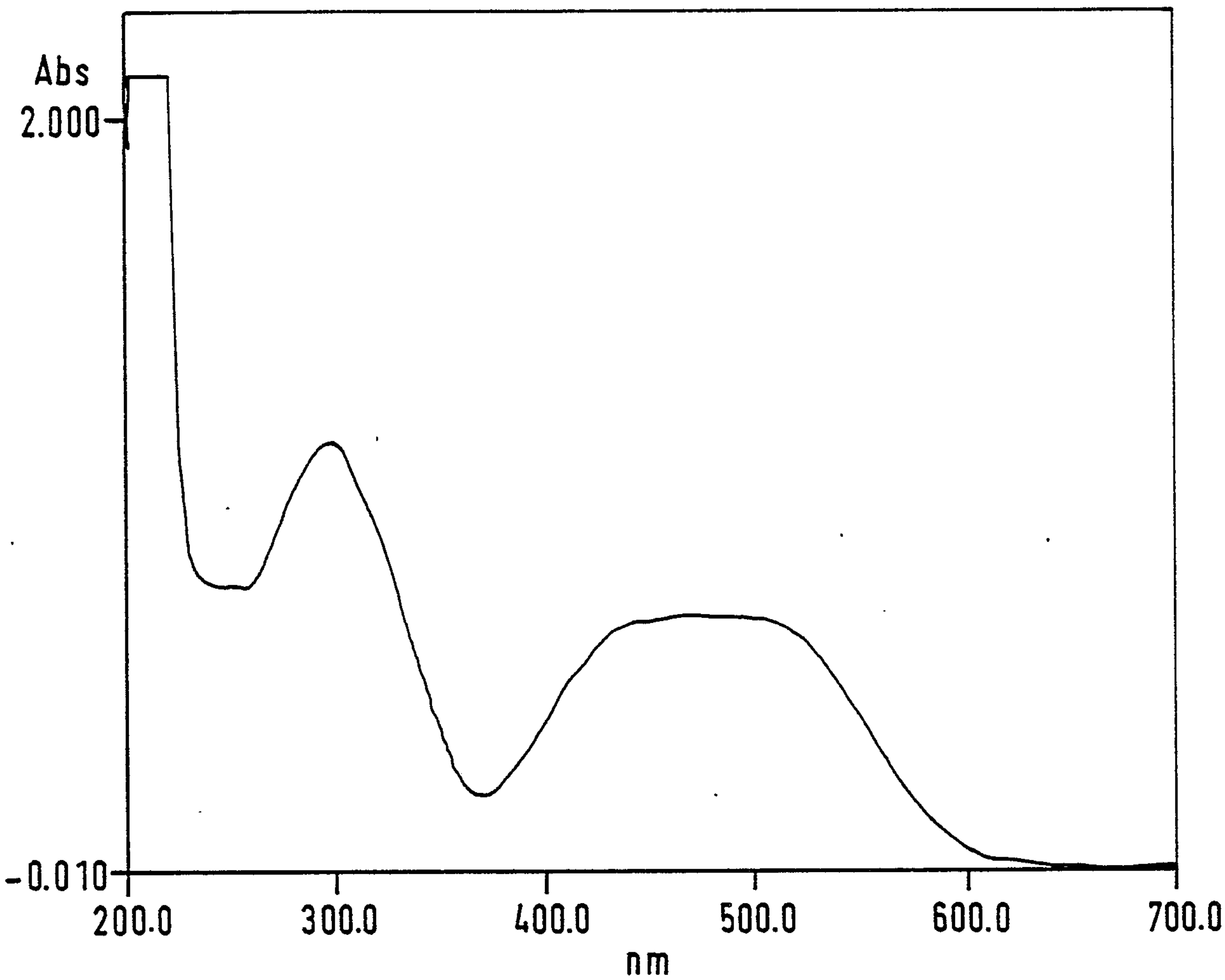

FIG. 3

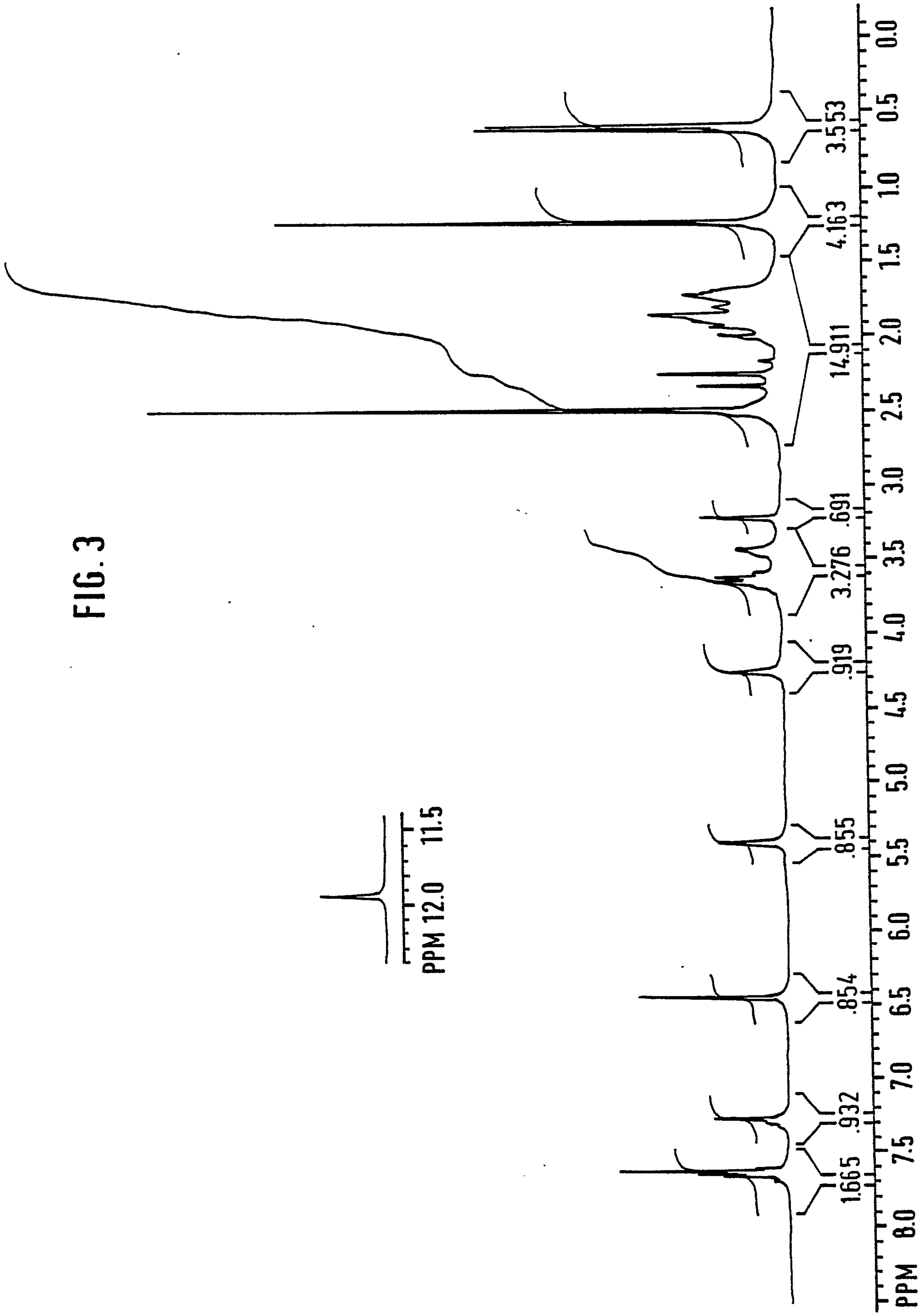
PPM 12.0
11.5
3.553
4.163
14.911
.691
3.276
.919
.855
.854
.932
1.665
PPM 8.0
7.5
7.0
6.5
6.0
5.5
5.0
4.5
4.0
3.5
3.0
2.5
2.0
1.5
1.0
0.5
0.0

FIG. 4

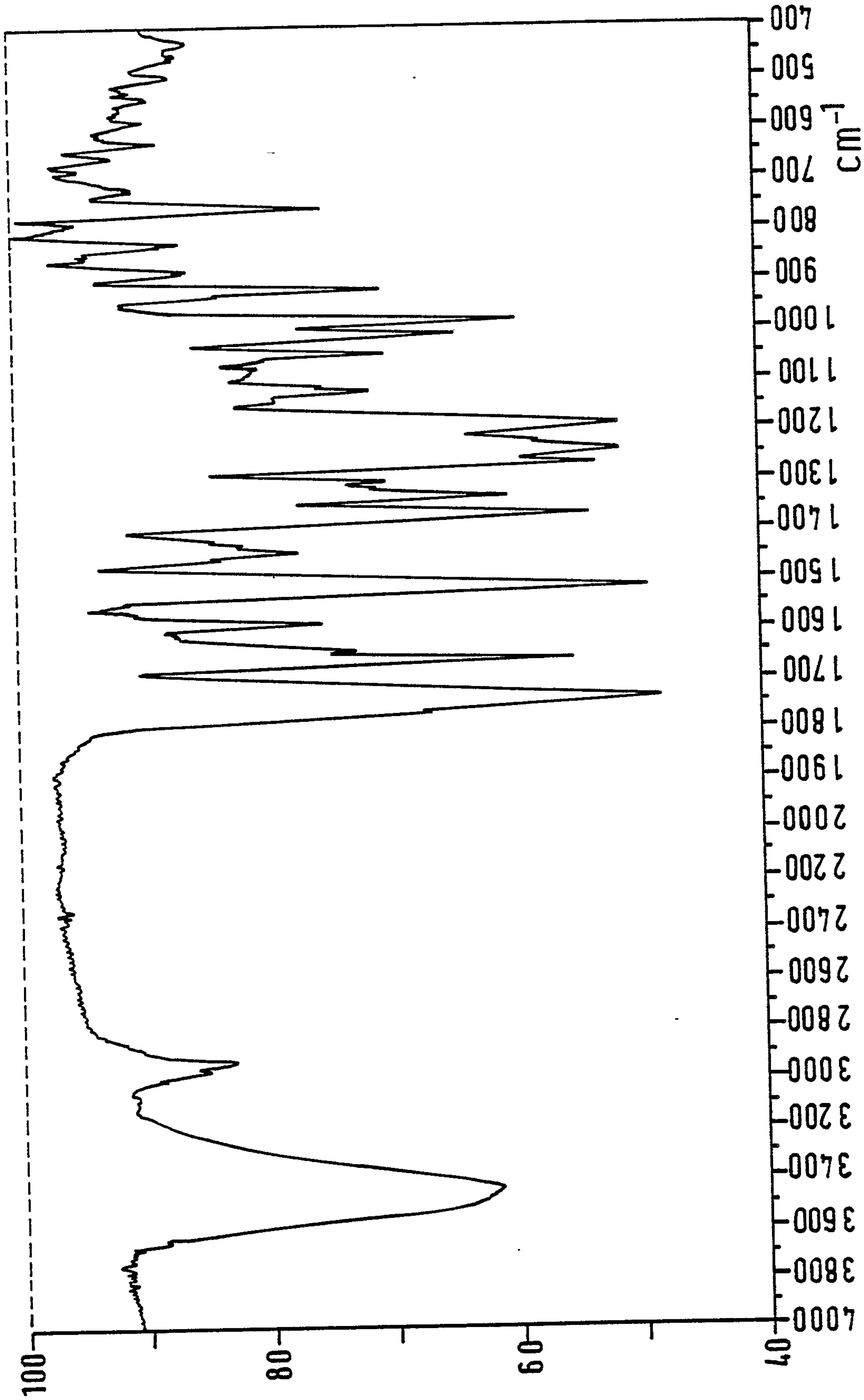

100
80
60
40
4 000
3 800
3 600
3 400
3 200
3 000
2 800
2 600
2 400
2 200
2 000
1 900
1 800
1 700
1 600
1 500
1 400
1 300
1 200
1 100
1 000
900
800
700
600
500
400
cm-1

FIG. 5

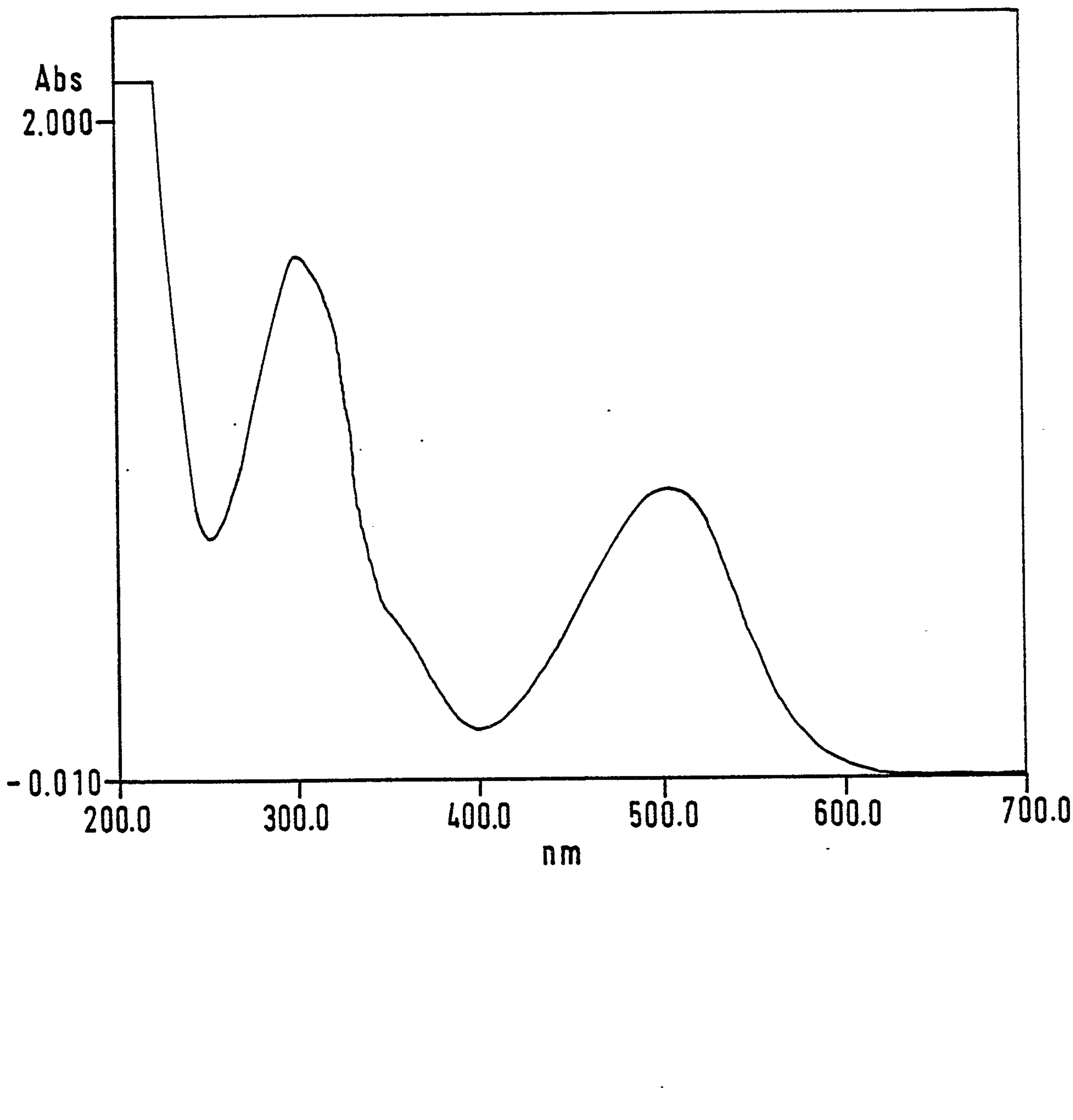

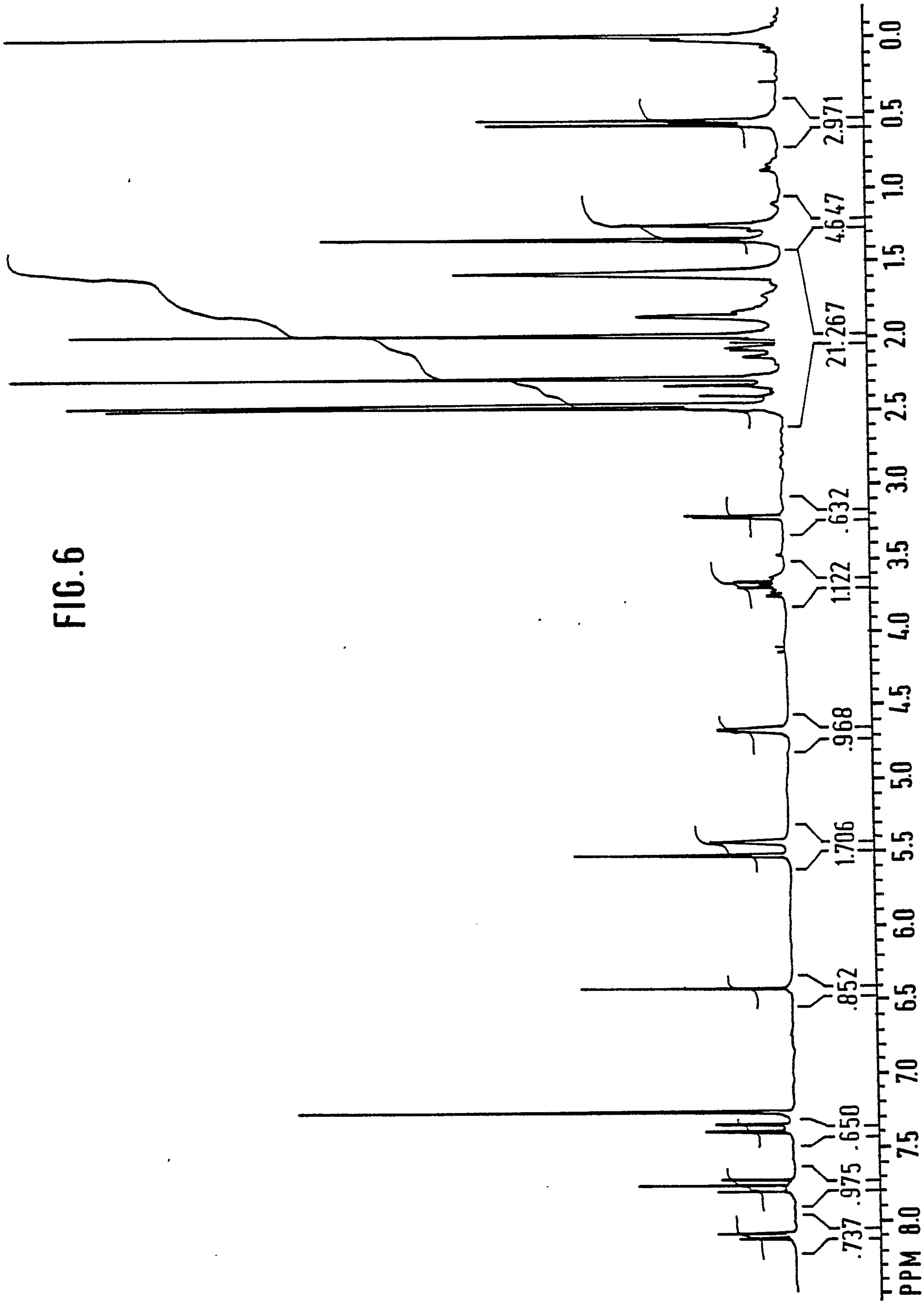
PPM
8.0
7.5
7.0
6.5
6.0
5.5
5.0
4.5
4.0
3.5
3.0
2.5
2.0
1.5
1.0
0.5
0.0
.737
.975
.650
.852
1.706
.968
1.122
.632
21.267
4.647
2.971

FIG.6